# EUROPÄISCHE PATENTSCHRIFT

(11) **EP 1 072 281 B1**
(45) Veröffentlichungstag und Bekanntmachung des Hinweises auf die Patenterteilung: **10.03.2004**
(21) Anmeldenummer: 00250237.5
(22) Anmeldetag: 13.07.2000
(51) Int. Cl.: A61M 25/01

(54) **Katheter mit Abstandssensor**
Catheter with distance sensor
Cathéter avec capteur de distance

(30) Priorität: 30.07.1999 DE 19936904
(43) Veröffentlichungstag der Anmeldung: 31.01.2001
(73) Patentinhaber: BIOTRONIK Mess- und Therapiegeräte GmbH & Co Ingenieurbüro Berlin, 12359 Berlin (DE)
(72) Erfinder: Kranz, Curt, 14163 Berlin (DE); Schaldach, Max, 91054 Erlangen (DE)
(74) Vertreter: Eisenführ, Speiser & Partner

(56) Entgegenhaltungen:
- EP-A- 0 284 055
- DE-A- 3 707 787
- FR-A- 2 732 225
- US-A- 4 930 494

## Beschreibung

Die Erfindung betrifft einen Katheter insbesondere für intravaskuläre Anwendungen im allgemeinen Sinn. In dem hier verstandenen allgemeinen Sinn sollen unter den Begriff Katheter beispielsweise auch durch Blutgefäße hindurch in ein Herz einführbare Elektrodenleitungen für Herzschrittmacher, Ablationskatheter und auch Führungsdrähte fallen. Führungsdrähte dienen insbesondere als Hilfsmittel zum Einführen von Kathetern im engeren Sinn oder von Herzschrittmacherelektrodenleitungen durch Blutgefäße des menschlichen Körpers hindurch.

Es sind eine Vielzahl solcher Katheter, beispielsweise Ablationskatheter und Führungsdrähte bekannt, die am proximalen Ende mit mechanischen Stellmitteln versehen sind, um das distale Ende durch entsprechende manuelle Betätigung der Stellmittel in eine gewünschte Richtung zu deflektieren. Auf diese Weise kann der Führungdraht manuell auch durch verwinkelte Blutgefäße hindurch eingeführt werden. Der einmal eingeführte Führungsdraht dient anschließend als Hilfsmittel zum Einführen eines Katheters oder einer Elektrodenleitung. Der Katheter kann dabei abschnittsweise auf den Führungsdraht aufgeclipt werden. Alternativ kann der Katheter auch über den Führungsdraht geschoben werden. Dazu weist ein solcher Katheter ein entsprechendes Lumen auf.

Ein von Hand steuerbarer Ablationskatheter ist beipielsweise aus der US 5,273,535 (nächstliegen der Stand der Technik) bekannt. Der Katheter besteht im wesentlichen aus einem Griffelement, das in einen Katheterschaft übergeht, von dem aus eine flexible Führungsröhre abgeht, die in eine Katheterspitze mit integrierter Elektrode ausläuft. An dem Katheterschaft gegenüberliegenden Ende des Griffelements befindet sich eine elektrische Leitung, über welche die Elektrode mit elektrischer Energie versorgt wird.

Innerhalb der flexiblen Führungsröhre verlaufen zwei an der Katheterspitze befestigte Zugdrähte. Die Zugdrähte sind derart angeordnet, daß durch Ziehen an dem einen oder an dem anderen Zugdraht eine Deflektion der Katheterspitze in eine erste Richtung oder einer dieser Richtung entgegengesetzten Richtung möglich ist. Eine Druckkraft wird durch die Zugdrähte nicht übertragen. Durch die Deflektion der Katheterspitze und eine zusätzliche Verdrehung der Führungsröhre mittels des Griffelements kann der Katheter dem kurvigen Verlauf eines Körperhohlorgans folgen kann, ohne eine Verletzung desselben zu verursachen.

Die Zugdrähte sind über im Griffelement untergebrachte Getriebemittel wechselnd ziehend bewegbar, die durch ein Stellelement manuell bedient werden. Das Stellelement ist hier als Stellrad ausgeführt. Die Getriebemittel wandeln die Drehbewegung des Stellrades in die ziehende Längsbewegung für die Zugdrähte um. Die Getriebemittel bestehen aus einer dem Stellrad gegenüber koaxial und ortsfest angeordneten Welle, an die je ein Zugdraht von beiden Seiten her anliegt. Die beiden Zugdrähte sind an einem Scheitelpunkt der Welle befestigt. Wird nun beispielsweise eine Linksdrehung am Stellrad ausgeführt, so dreht sich die Welle ebenfalls nach links und ein Draht wird dem zurückgelegten Winkel entsprechend auf die Welle aufgewickelt und dadurch gezogen. Der andere Zugdraht wird entsprechend entlastet. Es kommt zu einer Deflektion der Katheterspitze. Eine entgegengesetzte Deflektion wird in analoger Weise über eine Rechtsdrehung am Stellrad erzielt. Der Grad der Deflektion ist bei dieser Konstruktion durch den Durchmesser der Welle festgelegt und wegen der baulichen Randbedingungen recht gering.

Andere von Hand steuerbare Katheter gehen den US 5,254,088 und US 5,364,351 hervor. Von diesen zeigt die US 5,245,088 verschiedene Varianten von Kathetern, mit jeweils einem Paar vom in einem Lumen des Katheters angeordneten Steuerdrähten, die nahe dem distalen Ende des Katheters miteinander verbunden sind, so daß sich durch eine Relativbewegung der Steuerdrähte zueinander in deren axialer Richtung eine seitliche Auslenkung des distalen Endes des Katheters erzielen läßt. Die radiale Richtung, in der diese Auslenkung erfolgt, läßt sich dadurch einstellen, daß die Steuerdrähte bezüglich des übrigen Katheters um eine gemeinsame Längsachse rotiert werden. Relative axiale Verschiebung der Steuerdrähte zueinander und deren Rotation bezüglich der Katheterhülle lassen sich mittels eines Handsteuergerätes am proximalen Ende des Katheters einstellen.

Ausgehend von diesem Stand der Technik ist es Ziel der Erfindung, einen Katheter anzugeben, der sich einfacher als bekannte Katheter insbesondere durch Blutgefäße des menschlichen Körpers hindurch bis zu einem gewünschten Ort, beispielsweise in ein Herz, einführen läßt.

Erfindungsgemäß wird diese Aufgabe durch einen Katheter mit wenigstens einem, am distalen Endes des Katheters angeordneten Sensor, welcher ausgebildet ist, ein vom Abstand des Sensors zur Gefäßwand abhängiges Abstandssignal aufzunehmen und mit Steuermitteln, die mit dem Sensor zur Übernahme des Abstandssignals verbunden sind.

Indem ein Abstandssignal unmittelbar am distalen Ende des Katheters aufgenommen wird, steht an den Steuermitteln ohne zusätzliche, beispielsweise extrakoporale Hilfsmittel eine Information über die Lage des distalen Endes des Katheters in beispielsweise einem Blutgefäß zur Verfügung. Dasdementsprechnde Abstandssignal kann durch entsprechende Gestaltung der Steuermittel in jeder gewünschten Form verarbeitet und insbesondere zur Steuerung der Deflektion der Katheterspitze, sei es manuel, sei es automatisch, genutzt werden. Die Erfindung schließt somit die Erkenntnis ein, die Annäherung des distalen Endes des Katheters sensorisch zu erfassen und ein entsprechendes Signal für die Steuerung der Deflektion des Katheters zur Verfügung zu stellen, so daß das distale Ende des Katheters bei Annäherung an eine Gefäßwand derart deflektiert werden kann, daß es sich von der Gefäßwand entfernt.

In diesem Zusammenhang wird ein Katheter besonders bevorzugt, der sich durch Aktuatoren auszeichnet, die mit den Steuermitteln in Wirkverbindung stehen und die zum Deflektieren des Katheters ausgebildet sind, wobei die Steuermittel ausgebildet sind, auf ein Abstandssignal der Sensoren hin ein entsprechendes Steuersignal für die Aktuatoren zu generieren und an diese weiterzuleiten, um die Aktuatoren so anzusteuern, daß sich das distale Ende des Katheters infolge entsprechender Deflektion durch die Aktuatoren von der Gefäßwand entfernt. Ein solcher Katheter bietet den großen Vorteil, daß er mit seinem distalen Ende von sich aus, wann immer möglich, einen Abstand zu den Gefäßwänden einhält und daher ohne aufwendige Beobachtung und manuelle Steuerung in ein Blutgefäß ein- und durch es hindurchgeführt werden kann. Die Erfindung schließt mithin den Gedanken ein, ein Sensoren für das Abstandssignal mit entsprechenden Steuermittel und Aktuatoren zur Deflektion des Katheters zu einem automatisch arbeitenden System zu kombinieren.

Die Aktuatoren oder die Steuermittel oder beide können grundsätzlich sowohl am distalen als auch am proximalen Ende des Katheters angeordnet sein. Für die Anordnung am distalen Ende eignen sich beispielsweise piezoelektrische Aktuatoren. In einer alternativen Ausgestaltung der Erfindung kann ein Katheter auch so gestaltet sein, daß die von dem Sensor ermittelten Abstandssignale am proximalen Ende angezeigt werden, um die bekannte Steuerung des Katheters mit manuellen Einstellmitteln zum Deflektieren des Katheters zu vereinfachen. Dementsprechend wird auch ein Katheter bevorzugt, der Anzeigemittel umfaßt, die zur Ansteuerung durch die Steuermittel mit diesen verbunden sind.

Vorzugsweise sind am distalen Ende des Katheters mindestens drei Sensoren radial verteilt angeordnet. Auf diese Weise ist es möglich, eine Information über die Richtung der Annäherung des Katheters an eine Gefäßwand zu erlangen und der Annäherung durch entsprechende Ansteuerung der Aktuatoren entgegenzuwirken.

Außerdem wird ein Katheter bevorzugt, dessen Sensor ein optischer Sensor ist bzw. dessen Sensoren optische Sensoren umfassen. Besonders bevorzugt ist dabei eine Variante eines Katheters, bei der der Sensor oder die Sensoren Lichtleiter umfassen, die am distalen Ende des Katheters enden. In diesem Falle kann das Ende eines Lichtleiters selbst als Sensor für auf das Ende des Lichtleiters treffendes Licht dienen. Dementsprechend wird ein Katheter bevorzugt, bei dem mindestens einer der Lichtleiter zur Aufnahme von Licht am distalen Ende des Katheters und Weiterleiten des aufgenommenen Lichtes an die Steuermittel ausgebildet ist. Das aufgenommene Licht stellt dabei das Abstandssignal dar. Der Katheter weist vorzugsweise einen weiteren Lichtleiter auf, der zur Abgabe von Licht am distalen Ende des Katheters ausgebildet ist und vorzugsweise am proximalen Ende des Katheters mit einer infrarotes Licht abgebenden Lichtquelle verbunden ist. Letzterer Variante des Katheters liegt die Erkenntnis zugrunde, daß Blut im Infrarotbereich zwischen etwa 600 und 650 nm eine Transmission von ca. 90% aufweist und somit "durchsichtig" ist. So ist es möglich, über den einen Lichtleiter infrarotes Licht am distalen Ende des Katheters austreten zu lassen und über andere Lichtleiter die Stärke des von den Gefäßwänden reflektierten infraroten Lichtes als Abstandssignal zum proximalen Ende des Katheters zurückzuführen, wo aus dem Abstandssignal entweder Anzeigewerte oder Steuersignale für entsprechende Aktuatoren abgeleitet werden.

Im Zusammenhang mit optischen Sensoren werden Steuermittel bevorzugt, die zum Verarbeiten optischer Signale ausgebildet sind.

Alternativ zur letztgenannten Variante können auch Steuermittel vorgesehen werden, die zur Verarbeitung elektrischer Signale ausgebildet sind. Dementsprechend zeichnen sich alternativ bevorzugte Katheter durch einen Sensor bzw. mehrere Sensoren aus, die elektrische Signale abgeben. Solche Sensoren können kapazitive Näherungssensoren sein, die ihre Kapazität mit Annäherung an eine Gefäßwand ändern. Aus der Dämpfung eines elektrischen Wechselstromsignals kann dann ein Abstandssignal abgeleitet werden.

Ein alternativ bevorzugter elektrischer Sensor umfaßt Impedanzerfassungsmittel mit wenigstens zwei Elektroden, die zum Erfassen der Impedanz von zwischen den Elektroden befindlichem Blut ausgebildet sind. Ein solcher Sensor nutzt den physikalischen Effekt, daß Blut je nach Strömungszustand, laminar oder turbulent, der sich in Abstand von der Entfernung zur Gefäßwand einstellt, einen unterschiedlichen Leitwert hat, so daß sich auf diese Weise ein Abstandssignal ableiten läßt.

Eine weitere Alternative für elektrische Sensoren stellen beheizbare Thermistoren dar, deren elektrischer Widerstand sich mit der Temperatur ändert. Aufgrund der Beheizung der Thermistoren hängt deren Temperatur davon ab, wieviel Wärme vom distalen Ende des Katheters an das Blut abgegeben wird. Diese Wärmemenge hängt jedoch von der Strömungsgeschwindigkeit des Blutes relativ zum Katheter ab. Dabei ist die Strömungsgeschwindigkeit des Blutes in Nähe der Gefäßwand kleiner als mit Abstand von der Gefäßwand. Dementsprechend wird der Thermistor mit größerer Entfernung von der Gefäßwand stärker gekühlt, so daß das der Temperatur des Thermistors entsprechende Signal als Abstandssignal dienen kann.

Im Falle eines mit Aktuatoren und Steuermitteln versehenen Katheters sind bevorzugt Handsteuermittel vorgesehen, die mit den Aktuatoren in Wirkverbindung stehen und derart ausgebildet sind, daß sie ein Deflektieren des Katheters in vorgebbarer Weise unabhängig von den Abstandssignalen erlauben, welche von den Sensoren an die Steuermittel geleitet werden. Auf diese Weise kann die automatische Steuerung des Katheters durch eine Handsteuerung gesteuert werden, um beispielsweise bei Gefäßverzweigungen gezielt von Hand eine Richtung vorgeben zu können.

Die Erfindung soll nun anhand von Ausführungsbeispielen mit Hilfe der Figuren näher erläutert werden. Diese zeigen:
- Figur 1: einen erfindungsgemäßen Katheter zur Handsteuerung;
- Figur 1a: einen Querschnitt durch den Katheter in Figur 1;
- Figur 2: einen zur automatischen Steuerung geeigneten Katheter;
- Figur 3: einen für eine Hand- und eine automatische Steuerung geeigneten Katheter;
- Figur 3a: einen Querschnitt durch den Katheter in Figur 3;
- Figur 4: das distale Ende eines der Katheter aus den Figuren 1 bis 3 mit einer ersten Ausführungsform eines elektrischen Abstandssensors;
- Figur 5: das distale Ende eines der Katheter aus den Figuren 1 bis 3 mit einer alternativen Ausführungsform eines elektrischen Abstandssensors;
- Figur 6: das distale Ende eines der Katheter aus den Figuren 1 bis 3 mit einem optischen Abstandssensor;
- Figur 6a: eine Aufsicht auf die Katheterspitze des Katheters aus Figur 6 und
- Figur 7: einen Katheter ähnlich Figur 1 mit einer Anzeigeeinheit für ein quasiendoskopisch gewonnenes Bild.

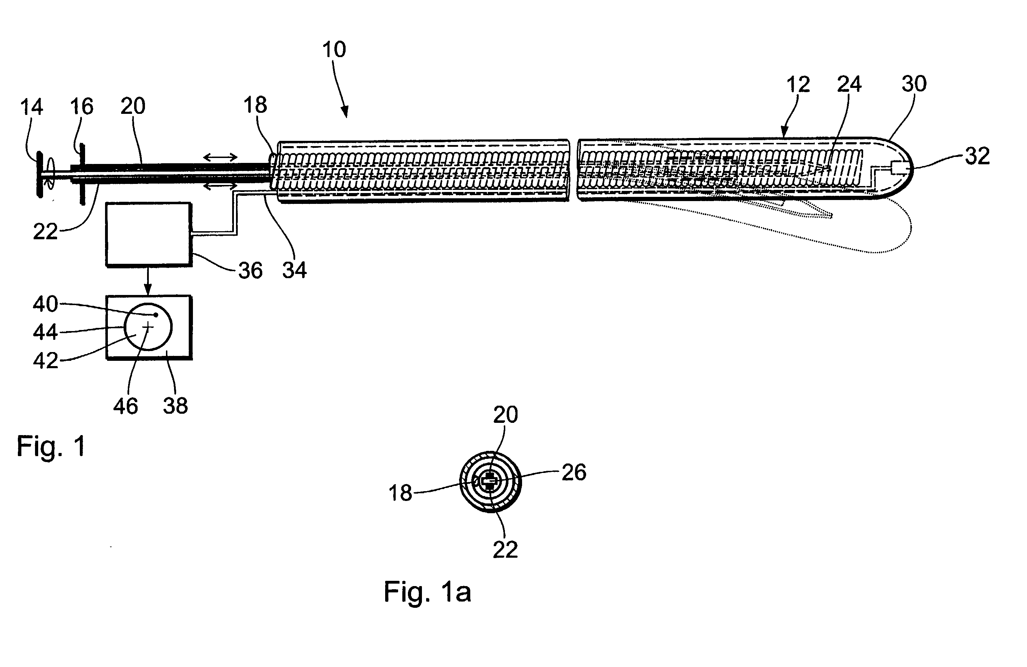

Figur 1 zeigt einen Katheter 10, dessen distales Ende 12 in beliebiger radialer Richtung durch Deflektion seitlich auslenkbar ist. Diese Auslenkung erfolgt beispielsweise nach dem aus der US 5,254,088 bekannten Prinzip. Der Katheter 10 weist dazu an seinem proximalen Ende Handsteuermittel 14 und 16 auf, die mit einer ein Lumen einschließenden, an ihrem distalen Ende flexiblen Spiralhülle 18 sowie zwei in dem Lumen der Spiralhülle 18 geführten Steuerdrähten 20 und 22 verbunden sind. Die Handsteuermittel 16 sind in bekannter Weise derart mit den beiden Steuerdrähten 20 und 22 verbunden, daß diese in axialer Richtung relativ zueinander verschiebbar sind. An ihrem distalen Ende 24 sind die beiden Steuerdrähte 20 und 22 miteinander verbunden. Durch das axiale Verschieben der Steuerdrähte relativ zueinander kommt es zu einer seitlichen Ausbiegung der Spiralhülle 18 und damit des Katheters 10 im flexiblen Bereich der Spiralhülle 18 am distalen Ende 12 des Katheters 10.

Die Spiralhülle 18 ist in dem Katheter 10 relativ zum Katheter 10 rotierbar angeordnet. Durch eine Rotationsbewegung der Spiralhülle 18 mit den darin geführten Steuerdrähten 20 und 22 bezüglich des Katheters 10 kann die radiale Richtung der seitlichen Auslenkung beim Deflektieren des distalen Katheterendes 12 bestimmt werden. Dazu ist in der Spiralhülle 18 ein Flachband 26 vorgesehen, das das Lumen in der Spiralhülle 18 in zwei Hälften teilt, die jeweils einen der Führungsdrähte 20 und 22 führen. An dem Flachband 26 greift am distalen Ende das Handsteuermittel 14 derart an, daß das Flachband 26 und mit ihm die Führungsdrähte 20 und 22 relativ zum übrigen Katheter 10 rotierbar sind. Die Spiralhülle 18 kann, aber muß nicht notwendigerweise zusammen mit dem Flachband 26 in den Führungsdrähten 20 und 22 relativ zum übrigen Katheter rotiert werden. Entscheidend ist die Rotation der Führungsdrähte 20 und 22. Die Figur 1 a zeigt die Spiralhülle 18 mit den darin angeordneten Steuerdrähten 20 und 22 sowie dem Flachband 26 im Schnitt.

Im Bereich der Katheterspitze 30 an deren distalem Ende 12 ist ein Abstandssensor 32 angeordnet, der über eine als Bus dargestellte Signalverbindung 34 mit einer Steuereinheit 36 in Verbindung steht. Die Steuereinheit 36 steht ihrerseits mit einer Anzeigeeinheit 38 in Verbindung. Die Steuereinheit 36 generiert aus von dem Abstandssensor 32 empfangenen Abstandssignalen Steuersignale für die Anzeigeeinheit 38. Die aus den Abstandssignalen gewonnene Abstandsinformation bezüglich der relativen Lage der Katheterspitze 30 zwischen den Wänden beispielsweise eines Blutgefäßes wird durch die Anzeigeeinheit 38 derart dargestellt, daß ein Symbol 40 für die Katheterspitze 30 in einem Anzeigefeld 42 dargestellt wird, dessen äußere Begrenzung 44 die Gefäßwand des Blutgefäßes repräsentiert, in dem sich die Katheterspitze 30 befindet und das eine Markierung 46 zur Kennzeichnung der Mitte des Blutgefäßes enthält.

Mit Hilfe der über die Anzeigeeinheit 38 dargestellten Information kann die Katheterspitze 30 mittels der Handsteuerelemente 14 und 16 so gesteuert werden, daß sie sich möglichst immer in der Mitte des Gefäßes befindet, durch das die Katheterspitze 30 hindurchgeführt wird. Dazu muß das die Katheterspitze 30 repräsentierende Symbol 40 in dem Anzeigefeld 44 möglichst in der Nähe der die Gefäßmitte repräsentierenden Marke 46 gehalten werden. Beliebige Abweichungen können selbstverständlich jederzeitvon Hand eingestellt werden, um beispielsweise die Katheterspitze in Gefäßabzweigungen einführen zu können.

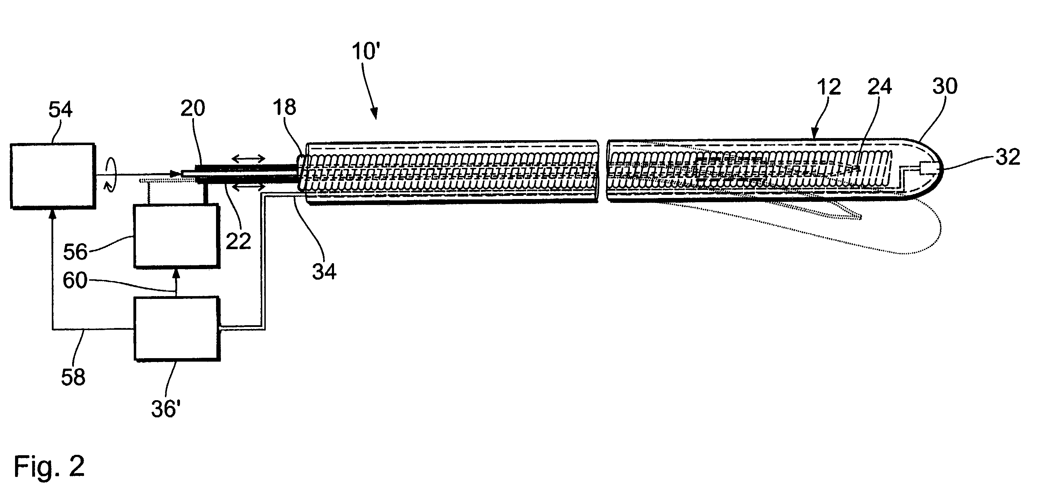

Figur 2 zeigt einen Katheter 10', der in wesentlichen Teilen dem in Figur 1 dargestellten entspricht. Die identischen Teile sind mit den gleichen Bezugszeichen wie in Figur 1 bezeichnet. Der wesentliche Unterschied zwischen dem Katheter 10' aus Figur 2 und dem Katheter 10 aus Figur 1 sind zwei mechanische Stellantriebe bzw. Aktuatoren 54 und 56 für die Rotation der Führungsdrähte 20 und 22 bezüglich des übrigen Katheters 10' und für die axiale Bewegung der Steuerdrähte 20 und 22 relativ zueinander. Die Steuereinheit 36' ist wie bei dem Katheter 10 aus Figur 1 mit dem Abstandssensor 32 über eine Signalverbindung 34 verbunden. Aus dem von dem Sensor 32 empfangenen Abstandssignal generiert die Steuereinheit 36 Steuersignale für die Aktuatoren 54 und 56, mit denen die Steuereinheit 36' über entsprechende Steuerleitungen 58 und 60 verbunden ist. Die Steuersignale für die Aktuatoren 54 und 56 werden anhand des Abstandssignals so generiert, daß die Katheterspitze 30 bei Annäherung an eine Gefäßwand durch die mittels der Aktuatoren 54 und 56 bewirkte Deflektion des distalen Endes 12 des Katheters 10' von der Gefäßwand wegbewegt. Der Katheter 10'stellt somit ein System dar, das sich ohne manuelle Steuerung in ein Blutgefäß ein- und durch dieses hindurchführen läßt.

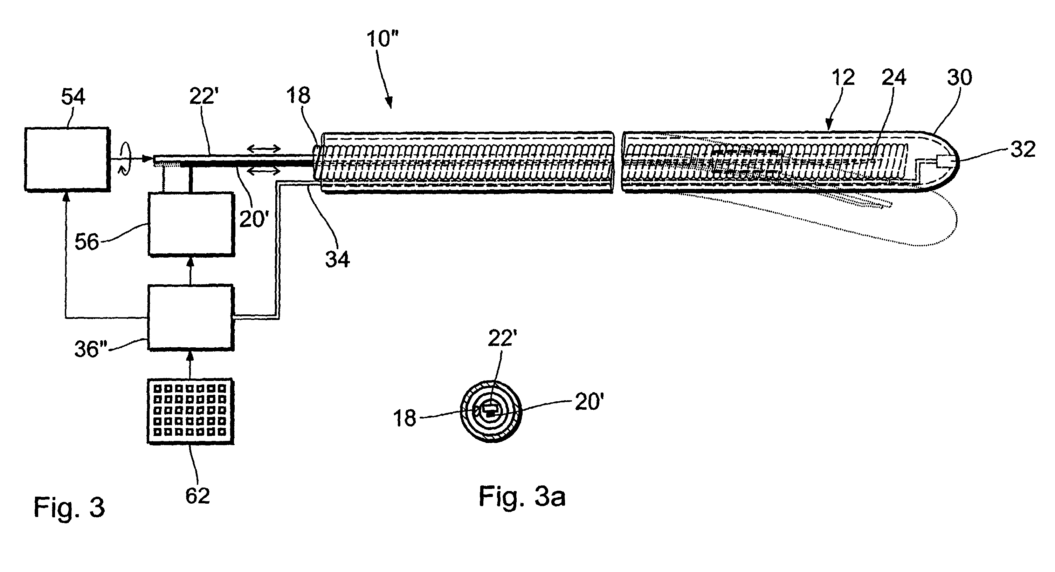

Figur 3 zeigt einen ähnlichen Katheter wie Figur 2. Zusätzlich zu den bereits in Figur 2 dargestellten Bestandteilen weist der Katheter 10' aus Figur 3 eine Handsteuereinheit 62 auf, mit der die aus dem Abstandssignal des Abstandssensors 32 gewonnenen Steuersignale für die Aktuatoren 54 und 56 übersteuert werden können, um die Spitze 30 des Katheters 10" von Hand gezielt auf eine Gefäßwand zuzudeflektieren, und die Katheterspitze 30 beispielsweise in eine dort befindliche Gefäßverzweigung einzuführen. Dazu ist die Handsteuereinheit 62 mit der Steuereinheit 36" verbunden. Außerdem ist in Figur 3 ein etwas anderer Aufbau der in dem Lumen der Spiralhülse 18 angeordneten Steuerdrähte 20' und 22' dargestellt. Diese sind, wie sich aus der Schnittansicht in Figur 3a ergibt, als Flachbänder ausgeführt und erübrigen somit die Verwendung eines zusätzlichen Flachbandes wie des Flachbandes 26 aus den Figuren 1 und 2.

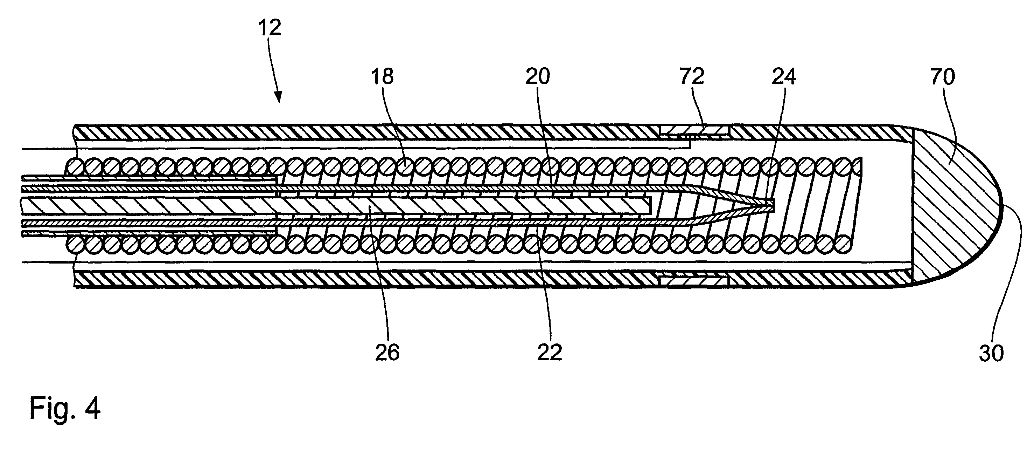

Der Abstandssensor 32 der Katheter 10, 10' und 10" kann beispielsweise ein kapazitiver Abstandssensor sein. Alternative Abstandssensoren sind in den Figuren 4 bis 6 dargestellt. Das in Figur 4 abgebildete distale Ende 12 eines Katheters 10 ist mit zwei Elektroden 70 und 72 versehen, von denen die Elektrode 70 als Tipelektorde in der Katheterspitze 30 ausgebildet ist, während die Elektrode 72 eine Ringelektrode ist. Die Elektroden sind über Signalleitungen mit der in Figur 4 nicht dargestellten Steuereinheit 36, 36', 36" verbunden. Die Elektroden 70 und 72 können zur kapazitiven Abstandsmessung oder zur Messung der Impedanz des zwischen den Elektroden befindlichen Blutes verwendet werden. Auf diese Weise läßt sich ein Abstandssignal auf die weiter vorne beschriebene Art gewinnen.

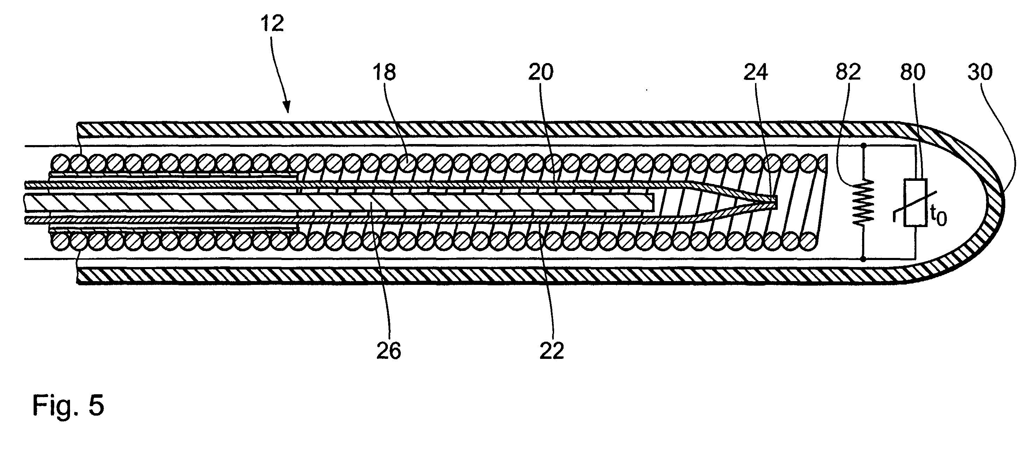

In Figur 5 ist der Abstandssensor 32 als temperaturabhängiger Widerstand, also als Thermistor 80 ausgebildet. Außerdem ist ein Heizelement 82 vorgesehen, um die Katheterspitze 30 zu beheizen. In der vorne beschriebenen Weise kann aus der Kühlung des Thermistors 80 ein Signal für den Abstand der Katheterspitze 30 zur Gefäßwand abgeleitet werden.

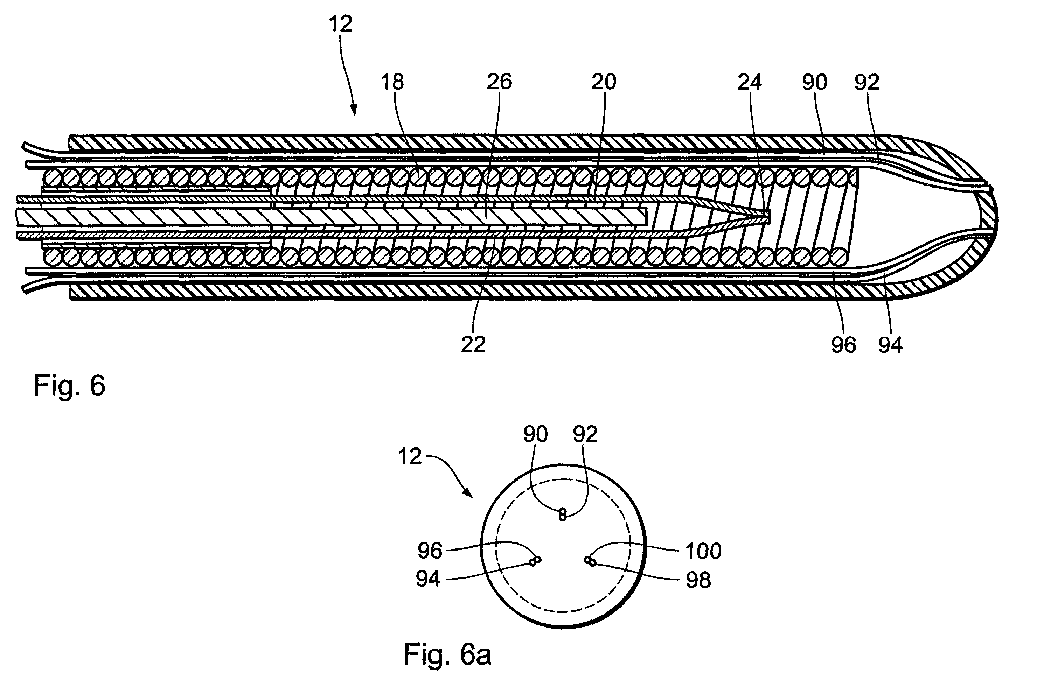

In der in Figur 6 dargestellten Ausführungsform ist der Abstandssensor 32 ein optischer Sensor. Der Abstandssensor 32 wird von sechs Lichtwellenleitern 90, 92, 94, 96, 98 und 100 gebildet, die jeweils paarweise einander zugeordnet sind. Die Paare von Lichtwellenleitern sind gleichmäßig auf einer Umfangslinie im Bereich der Katheterspitze 30 verteilt, so daß jeweils einer der Lichtwellenleiter eines Paares bevorzugt Licht aufnimmt, der auf einen Sektor der Katheterspitze fällt, der dem jeweiligen Lichtwellenleiterpaar nahe ist. Diese Anordnung der Enden der Lichtwellenleiter 90, 92, 94, 96, 98 und 100 ist der Aufsicht auf die Katheterspitze 30 in Figur 6a zu entnehmen.

Von den paarweise einander zugeordneten Lichtwellenleitern dient einer der Aufnahme von Lichtsignalen und deren Weiterleitung an eine zur Bearbeitung optischer Signale geeignete Steuereinheit. Der jeweils andere einem Lichtwellenleiterpaar zugeordnete Lichtwellenleiter ist mit einer nicht dargestellten Infrarotlichtquelle am proximalen Ende des Katheters 10 verbunden und leitet Licht aus dieser Lichtquelle zum distalen Ende des Katheters 10, wo es am Ende des entsprechenden Lichtwellenleiters austritt und die entsprechende Umgebung der Katheterspitze 30 beleuchtet. Von Gefäßwänden reflektiertes Infrarotlicht wird jeweils von dem anderen Lichtwellenleiter eines Lichtwellenleiterpaares aufgenommen und an die Steuereinheit übertragen, wo das Lichtsignal mit denjenigen der beiden anderen Licht empfangenden Lichtwellenleiter der übrigen beiden Lichtwellenleiterpaare verglichen wird. Da Blut in einem Wellenlängenbereich zwischen 600 und 650 nm eine Transmission in der Nähe von 90% hat, hängt die Stärke des empfangenen Lichtsignals davon ab, wie nahe sich ein Lichtwellenleiterpaar an einer Gefäßwand befindet. Eine stärkere Reflektion bedeutet eine größere Nähe zur Gefäßwand. Zum Vergleich der drei auf die beschriebene Weise gewonnenen Lichtsignale in der Steuereinheit kann leicht ermittelt werden, in welcher auf den Katheter bezogenen radialen Richtung sich die Katheterspitze 30 einer Gefäßwand nähert und über die Aktuatoren entsprechend gegengesteuert werden. Die drei Licht aufnehmenden Lichtwellenleiter der der Steuereinheit zugehenden Lichtsignale bilden dabei zusammen das Abstandssignal.

Eine alternative Ausführung des Abstandssensors 32, die ebenfalls mit Lichtwellenleitern und Infrarotlicht arbeitet, weist nur zwei Lichtwellenleiter auf, von denen einer Licht emittiert und der andere an der Spitze des Katheters 10 von den Seiten einfallendes Licht aufnimmt und der Steuereinheit zuführt. Aufgrund der nicht hundertprozentigen Transmission des Blutes ist das Lichtsignal als Abstandssignal dann am schwächsten, wenn sich die Katheterspitze 30 in der Mitte des Gefäßes befindet.

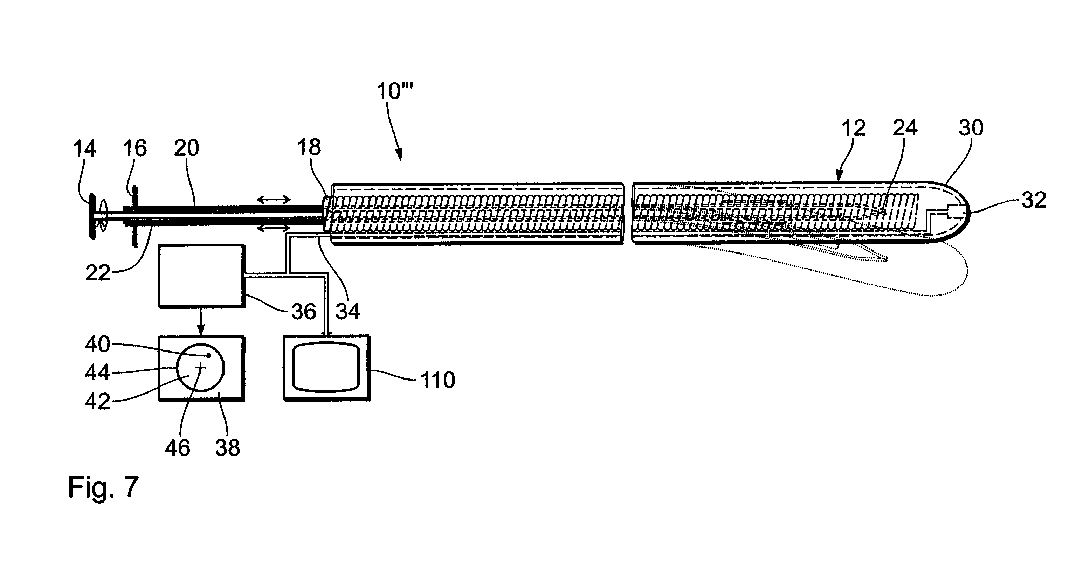

Der Katheter 10''' in Figur 7 weist zusätzlich zu dem in Figur 1 abgebildeten Katheter 10 eine Anzeigeeinheit 110 auf, die mit dem Sensor 32 verbunden ist. Der Sensor 32 ist dabei als optischer bildaufnehmender Sensor ausgebildet. Das von dem Sensor 32 aufgenommene Bild wird durch die Anzeigeeinheit 110 dargestellt. Der Katheter 10''' wird dadurch praktisch zu einem endoskopartigen Gerät. Die mit Hilfe des Sensors 32 gewonnene und durch die Anzeigeeinheit 110 dargestellte Information kann insbesondere zur geeigneten Plazierung des Katheters verwendet werden. Es ist mit einem solchen Katheter möglich, geeignete Elektrodenplazierungen zu suchen oder geeignete Ablationsorte, falls es sich um einen Ablationskatheter handelt.

## Patentansprüche

1. Katheter (10, 10', 10", 10''') insbesondere zum Einführen in Blutgefäße des menschlichen Körpers,
mit wenigstens einem, am distalen Ende (12) des Katheters (10, 10', 10", 10''') angeordneten Sensor (32), welcher ausgebildet ist, ein vom Abstand des Sensors (32) zur Gefäßwand abhängiges Abstandssignal aufzunehmen und mit Steuermitteln (36, 36', 36"), die mit dem Sensor zur Übernahme des Abstandssignals verbunden sind.

2. Katheter nach Anspruch 1, **gekennzeichnet durch** Aktuatoren (54, 56), die mit den Steuermitteln (36', 36") in Wirkverbindung stehen und die zum Deflektieren des Katheters (10', 10", 10''') ausgebildet sind,
wobei die Steuermittel (36', 36") ausgebildet sind, auf ein Abstandssignal des Sensors (32) ein entsprechendes Steuersignal für die Aktuatoren (54, 56) zu generieren und an diese weiterzuleiten, um die Aktuatoren (54, 56) so anzusteuern, daß sich das distale Ende (12) des Katheters (10', 10", 10''') infolge entsprechender Deflektion **durch** die Aktuatoren (54, 56) von der Gefäßwand entfernt.

3. Katheter nach Anspruch 1 oder 2, **gekennzeichnet durch** Anzeigemittel (38), die zur Ansteuerung **durch** die Steuermittel mit diesen verbunden sind.

4. Katheter nach einem der Ansprüche 1 bis 3, **dadurch gekennzeichnet, daß** am distalen Ende des Katheters mindestens 3 Sensoren (90, 94, 98) radial verteilt angeordnet sind.

5. Katheter nach einem der Ansprüche 1 bis 4, **dadurch gekennzeichnet, daß** der Sensor bzw. die Sensoren optische Sensoren umfassen.

6. Katheter nach Anspruch 5, **dadurch gekennzeichnet, daß** die Sensoren Lichtleiter (90, 94, 98) umfassen, die am distalen Ende (12) des Katheters enden.

7. Katheter nach Anspruch 6, **dadurch gekennzeichnet, daß** mindestens einer der Lichtleiter (92, 96, 100) zur Abgabe von Licht am distalen Ende des Katheters ausgebildet ist.

8. Katheter nach Anspruch 7, **dadurch gekennzeichnet, daß** der Lichtleiter (92, 96, 100) zum Abgeben von Licht am proximalen Ende des Katheters mit einer infrarotes Licht abgebenden Lichtquelle verbunden ist.

9. Katheter nach einem der Ansprüche 6 bis 8, **dadurch gekennzeichnet, daß** mindestens einer der Lichtleiter (90, 94, 98) zur Aufnahme von Licht am distalen Ende des Katheters und zum Weiterleiten des aufgenommenen Lichtes an die Steuermittel ausgebildet ist.

10. Katheter nach einem der Ansprüche 5 bis 9, **dadurch gekennzeichnet, daß** die Steuermittel zum Verarbeiten optischer Signale ausgebildet sind.

11. Katheter nach einem der Ansprüche 1 bis 4, **dadurch gekennzeichnet, daß** die Steuermittel zum Verarbeiten elektrischer Signale ausgebildet sind.

12. Katheter nach Anspruch 11, **dadurch gekennzeichnet, daß** der Sensor bzw. die Sensoren kapazitive Näherungssensoren umfassen.

13. Katheter nach Anspruch 11 oder 12, **dadurch gekennzeichnet, daß** der Sensor bzw. die Sensoren Impedanzerfassungsmittel mit wenigstens zwei Elektroden umfassen, die zum Erfassen der Impedanz von zwischen den Elektroden befindlichem Blut ausgebildet sind.

14. Katheter nach einem der Ansprüche 1 bis 13, **gekennzeichnet durch** Handsteuermittel (62), die mit den Aktuatoren (54, 56) in Wirkverbindung stehen und derart ausgebildet sind, daß sie ein Deflektieren des Katheters in vorgebarer Weise unabhängig von den Abstandssignalen erlauben, welche von dem Sensor (32) an die Steuermittel (36") geleitet werden.

15. Katheter nach einem der Ansprüche 1 bis 14, **gekennzeichnet durch** Anzeigemittel (110), die mit mindestens einem Sensor (32; 90, 94, 98) verbunden und zum Anzeigen der von dem Sensor (32; 90, 94, 98) aufgenommenen Informationen ausgebildet sind.

16. Katheter nach Anspruch 15, **dadurch gekennzeichnet, dass** die Anzeigeeinheit (110) mit optischen Sensoren (90, 94, 98) verbunden ist und zum Anzeigen mindestens eines von dem Sensor (32; 90, 94, 98) aufgenommenen Bildes ausgebildet ist.

## Claims

1. Catheter (10, 10', 10'', 10'''), in particular for insertion into a blood vessel of the human body, comprising at least one sensor (32) arranged at the distal end (12) of the catheter (10, 10', 10'', 10''') and designed to pick up a spacing signal dependent on the spacing of the sensor (32) from the vessel wall, and control means (36, 36', 36'') connected to the sensor for transferring the spacing signal.

2. Catheter according to claim 1, **characterised by** actuators (54, 56) operatively connected to the control means (36', 36'') and designed to deflect the catheter (10, 10', 10'', 10'''), the control means (36, 36'), in response to a spacing signal of the sensor (32), being designed to generate a corresponding control signal for the actuators (54, 56) and to transmit it thereto to control the actuators (54, 56) such that the distal end (12) of the catheter (10, 10', 10'', 10''') is distanced from the vessel wall owing to corresponding deflection by the actuators (54, 56).

3. Catheter according to claim 1 or 2, **characterised by** display means (38) connected to the control means for control thereby.

4. Catheter according to any of claims 1 to 3, **characterised in that** at least three sensors (90, 94, 98) are arranged in a radial distribution at the distal end of the catheter.

5. Catheter according to any of claims 1 to 4, **characterised in that** the sensor or sensors comprise optical sensors.

6. Catheter according to claim 5, **characterised in that** the sensors comprise optical fibres (90, 94, 98) which end at the distal end (12) of the catheter.

7. Catheter according to claim 6, **characterised in that** at least one of the optical fibres (92, 96, 100) is designed to output light at the distal end of the catheter.

8. Catheter according to claim 7, **characterised in that** the optical fibre (92, 96, 100) for outputting light at the proximal end of the catheter is connected to a light source emitting infrared light.

9. Catheter according to any of claims 6 to 8, **characterised in that** at least one of the optical fibres (90, 94, 98) is designed to receive light at the distal end of the catheter and to transmit the received light to the control means.

10. Catheter according to any of claims 5 to 9, **characterised in that** the control means is designed for processing optical signals.

11. Catheter according to any of claims 1 to 4, **characterised in that** the control means is designed for processing electrical signals.

12. Catheter according to claim 11, **characterised in that** the sensor or sensors comprise(s) capacitive proximity sensors.

13. Catheter according to claim 11 or 12, **characterised in that** the sensor or sensors comprise(s) impedance detection means having at least two electrodes for detecting the impedance of blood between the electrodes.

14. Catheter according to any of claims 1 to 13, **characterised by** manual control means (62) operatively connected to the actuators (54, 56) and designed such that it allows deflection of the catheter in a predeterminable manner independently of the spacing signals passed from the sensor (32) to the control means (36'').

15. Catheter according to any of claims 1 to 14, **characterised by** display means (110) connected to at least one sensor (32; 90, 94, 98) and designed for displaying information picked up by the sensor (32; 90, 94, 98).

16. Catheter according to claim 15, **characterised in that** the display unit (110) is connected to optical sensors (90, 94, 98) and is designed for displaying at least one image picked up by the sensor (32; 90, 94, 98).

## Revendications

1. Cathéter (10, 10', 10", 10'''), en particulier pour l'introduction dans des vaisseaux sanguins du corps humain, comportant au moins un détecteur (32) disposé à l'extrémité distale (12) du cathéter (10, 10', 10", 10''') qui est agencé pour recevoir un signal de distance dépendant de la distance du détecteur (32) à la paroi du vaisseau et des moyens de commande (36, 36', 36") qui sont reliés au détecteur pour prendre en charge le signal de distance.

2. Cathéter selon la revendication 1 **caractérisé par** des actuateurs (54, 56) qui sont en liaison opérationnelle avec les moyens de commande (36', 36") et qui sont agencés pour dévier le cathéter (10', 10", 10'''), où les moyens de commande (36', 36") sont agencés pour générer, en réponse à un signal de distance du détecteur (32), un signal de commande correspondant pour les actuateurs (54, 56) et pour le transmettre à ceux-ci, pour commander les actuateurs (54, 56) de telle manière que l'extrémité distale (12) du cathéter (10', 10", 10''') s'éloigne de la paroi du vaisseau par suite de la déviation correspondante due aux actuateurs (54, 56).

3. Cathéter selon la revendication 1 ou 2 **caractérisé par** des moyens indicateurs (38) qui, pour la commande par les moyens de commande, sont reliés à ceux-ci.

4. Cathéter selon l'une des revendications 1 à 3 **caractérisé en ce qu'**au moins 3 détecteurs (90, 94, 98) sont disposés radialement répartis à l'extrémité distale du cathéter.

5. Cathéter selon l'une des revendications 1 à 4 **caractérisé en ce que** le détecteur ou les détecteurs comprennent des détecteurs optiques.

6. Cathéter selon la revendication 5 **caractérisé en ce que** les détecteurs comprennent des fibres optiques (90, 94, 98) qui aboutissent à l'extrémité distale (12) du cathéter.

7. Cathéter selon la revendication 6 **caractérisé en ce qu'**au moins l'une des fibres optiques (92, 96, 100) est agencée pour délivrer de la lumière à l'extrémité distale du cathéter.

8. Cathéter selon la revendication 7 **caractérisé en ce que** la fibre optique (92, 96, 100) pour délivrer de la lumière est reliée à une source lumineuse délivrant de la lumière infrarouge à l'extrémité proximale du cathéter.

9. Cathéter selon l'une des revendications 6 à 8 **caractérisé en ce qu'**au moins l'une des fibres optiques (90, 94, 98) est agencée pour recevoir de la lumière à l'extrémité distale du cathéter et pour transmettre aux moyens de commande la lumière reçue.

10. Cathéter selon l'une des revendications 5 à 9 **caractérisé en ce que** les moyens de commande sont agencés pour traiter des signaux optiques.

11. Cathéter selon l'une des revendications 1 à 4 **caractérisé en ce que** les moyens de commande sont agencés pour traiter des signaux électriques.

12. Cathéter selon la revendication 11 **caractérisé en ce que** le détecteur ou les détecteurs comprennent des détecteurs de proximité capacitifs.

13. Cathéter selon la revendication 11 ou 12, **caractérisé en ce que** le détecteur ou les détecteurs comprennent des agents de détection d'impédance avec au moins 2 électrodes qui sont agencés pour détecter l'impédance du sang situé entre les électrodes.

14. Cathéter selon l'une des revendications 1 à 13 **caractérisé par** des moyens de commande manuelle (62) qui sont en liaison opérationnelle avec les actuateurs (54, 56) et qui sont agencés de telle manière qu'ils permettent une déviation du cathéter d'une manière qui peut être prédéterminée indépendamment des signaux de distance qui sont transmis par le détecteur (32) aux moyens de commande (36").

15. Cathéter selon l'une des revendications 1 à 14 **caractérisé par** des moyens d'affichage (110) qui sont reliés à au moins un détecteur (32 ; 90, 94, 98) et qui sont agencés pour afficher les informations reçues par le détecteur (32 ; 90, 94, 98).

16. Cathéter selon la revendication 15 **caractérisé en ce que** l'unité d'affichage (110) est reliée à des détecteurs optiques (90, 94, 98) et est agencée pour afficher au moins une image reçue par le détecteur (32 ; 90, 94, 98).
